# EUROPEAN PATENT SPECIFICATION

(11) **EP 4 401 673 B1**
(45) Date of publication and mention of the grant of the patent: **15.07.2026**
(21) Application number: 22765633.7
(22) Date of filing: 22.08.2022
(51) Int. Cl.: A61F 2/00, A61L 31/10, A61L 31/14, A61L 31/16, A61N 1/375

(54) **SURGICAL SYSTEM AND METHODS OF USE**
CHIRURGISCHES SYSTEM UND VERFAHREN ZUR VERWENDUNG
SYSTÈME CHIRURGICAL ET PROCÉDÉS D'UTILISATION

(30) Priority: 13.09.2021 US 202117473123
(43) Date of publication of application: 24.07.2024
(73) Proprietor: Medtronic, Inc., Minneapolis, Minnesota 55432 (US)
(72) Inventor: GOODMAN, Jonathan T., Minneapolis, Minnesota 55432 (US); CHEN, Sean, Minneapolis, Minnesota 55432 (US); SEETHAMRAJU, Kasyap V., Minneapolis, Minnesota 55432 (US); YUAN, Charlene X., Minneapolis, Minnesota 55432 (US); BAHULEKAR, Raman V., Minneapolis, Minnesota 55432 (US); KALSCHEUER, Joseph L., Minneapolis, Minnesota 55432 (US)
(74) Representative: Zimmermann & Partner Patentanwälte mbB
(86) International application number: PCT/IB2022/057848
(87) International publication number: WO 2023/037188

(56) References cited:
- US-A1- 2017 319 755
- JOHN TIMMONS: "Alginates as haemostatic agents: worth revisiting?", WOUNDS UK, vol. 5, no. 4, 1 January 2009 (2009-01-01), pages 122 - 125, XP055028955

## Description

### TECHNICAL FIELD

The present disclosure generally relates to anchorage devices and methods configured for anchoring an implantable medical device within a body, wherein the anchorage device comprises at least one hemostatic agent and at least one antibiotic that are configured to elute over time.

### BACKGROUND

Some known anchorage devices may be used to secure an implantable medical device within a body of a patient. The anchorage device and implantable medical device can be inserted into a desired location within the body of the patient. The anchorage device can be used to help anchor or support the implantable medical device to surrounding tissue. Some known anchorage devices are used to provide temporary support to tissue during a healing process. For example, some known anchorage devices can secure one portion of tissue to another portion of tissue.

Infection and bleeding are the most serious complications after surgery. The estimated increase in costs due to surgical site infections (SSIs) was $11,876 for SSls overall ($7003 for superficial and $25,721 for deep infections). However, within the current $6 billion hemostat market, there are few produces that can address this unmet need. It would therefore be desirable to stop or reduce the flow of blood at a surgical site while anchoring the implantable medical device to tissue. Some known anchorage devices are made at least partially from and/or are configured to release collagen to stop or reduce the flow of blood. However, the collagen used in such devices is a heavily processed version of collagen that has undergone milling, acid treatment, and has been pepsinized. The processing steps enable collagen to be readily solubilized and cast into films, but also results in a reduction of molecular weight and potentially denatures the protein structure. In particular this processing can affect collagen's triple helical peptides, which are a native sequence that binds to receptors on platelets helping stimulate platelet aggregation. This disclosure describes an improvement over these prior art technologies.

### SUMMARY

New anchorage devices and methods are provided to help anchor or support an implantable medical device to surrounding tissue. In one embodiment, in accordance with the principles of the present disclosure, a surgical device includes a substrate and a first coating that covers at least a portion of the substrate. The first coating comprises a first polymer. The first coating has antibiotics and/or other active pharmaceutical ingredients API(s) dispersed in the first polymer such that the first polymer releases the antibiotics and/or other APIs as the first polymer degrades. A second coating covers at least a portion of the first coating. The second coating comprises a second polymer. The second polymer comprises an alginate. The second coating has a hemostatic agent dispersed in the second polymer such that the second polymer releases the hemostatic agent as the second polymer degrades. The hemostatic agent is selected from the group consisting of epinephrine, tranexamic acid, chitosan and oxidized regenerated cellulose.

In one embodiment, in accordance with the principles of the present disclosure, a surgical device includes a substrate made from a first polymer. The substrate comprises antibiotics and/or other API(s) dispersed in the first polymer such that the first polymer releases the antibiotics and/or other API(s) as the first polymer degrades. A coating covers at least a portion of the substrate. The coating comprises a second polymer. The second polymer comprises an alginate. The second coating has a hemostatic agent dispersed in the second polymer such that the second polymer releases the hemostatic agent as the second polymer degrades. The hemostatic agent being selected from the group consisting of epinephrine, tranexamic acid, chitosan and oxidized regenerated cellulose.

In one embodiment, in accordance with the principles of the present disclosure, a surgical device includes a substrate and a first coating that covers at least a portion of the substrate. The first coating comprises a first polymer. The first coating has antibiotics and/or other API(s) dispersed in the first polymer such that the first polymer releases the antibiotics and/or other API(s) as the first polymer degrades. A second coating covers at least a portion of the first coating. The second coating comprises a second polymer. The second polymer comprises an alginate. The second coating has tranexamic acid agent dispersed in the second polymer such that the second polymer releases the tranexamic acid as the second polymer degrades. The substrate includes a mesh made from a plurality of fibers. The first coating directly engages the fibers such that the first coating extends 360 degrees about each of the fibers along at least a portion of a length of each of the fibers. The second coating directly engages the first coating such that the second coating extends less than 360 degrees about each of the fibers along at least a portion of the length of each of the fibers. The first polymer comprises a tyrosine-derived polyarylate. The alginate is selected from the group consisting of Sodium Alginate and Calcium Alginate. In some embodiments, the alginate has a molecular weight ranging from low to high (Viscosity of 1% solution of 10-2000cps) and a range of ratios in mannuronic acid to gluluronic acid. In some embodiments, the alginate can exist in natural or oxidized form. In some embodiments, esterified hydroxyl groups on Alginate are used.. The antibiotics are selected from the group consisting of rifampin and minocycline, and mixtures thereof.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description and appended claims.

### BRIEF DESCRIPTION OF THE DRAWINGS

The present disclosure will become more readily apparent from the specific description accompanied by the following drawings, in which:
FIG. 1 is a plan view of components of a surgical system, in accordance with the principles of the present disclosure;
FIG. 2 is a perspective view of one embodiment of a component of the surgical system shown in FIG. 1, in accordance with the principles of the present disclosure;
FIG. 3 is a detailed top, cross-sectional view of the component shown in FIG. 2;
FIG. 4 is a perspective view of the component shown in FIG. 2;
FIG. 5 is a side, cross-sectional view of the component shown in FIG. 2;
FIG. 6 is a side, cross-sectional view of the component shown in FIG. 2;
FIG. 7 is a side, cross-sectional view of the component shown in FIG. 2;
FIG. 8 is a perspective view of the component shown in FIG. 2;
FIG. 9 is a perspective view of the component shown in FIG. 2 in situ;
FIG. 10 is a detailed side, cross-sectional view of one embodiment of a component of the surgical system shown in FIG. 1, in accordance with the principles of the present disclosure;
FIG. 11 is a graph showing relative release rates of agents from the components shown in FIG. 2 and FIG. 10;
FIG. 12 is a perspective view showing one step of making one embodiment of a component of the surgical system shown in FIG. 1, in accordance with the principles of the present disclosure; and
FIG. 13 is a perspective view showing one step of making one embodiment of a component of the surgical system shown in FIG. 1, in accordance with the principles of the present disclosure.

### DETAILED DESCRIPTION

For the purposes of this specification and appended claims, unless otherwise indicated, all numbers expressing quantities of ingredients, percentages or proportions of materials, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding the numerical ranges and parameters set forth herein, the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

Post operative hematoma formation following the implantation of electronic implantable devices (EIDs) is a significant device related complication. It can result in increased patient pain, an increased risk of infection, and may necessitate surgical intervention. Essebag, V. Verma, A., et al. Clinically significant pocket hematoma increases long-term risk of device infection. Journal of The American College of Cardiology, 2016. The widespread use of anticoagulant and antiplatelet medications has been attributed to increasing the risk of pocket related hematomas. The reported frequency of EID-related hematomas varies, but it is believed to be 2-5%. Chen, Y., Li, Y., Gao, M. et al. Incidence of pocket hematoma after electrophysiological device placement: dual antiplatelet therapy versus low-molecular weight heparin regimen. Journal of Geriatric Cardiology, 2014. Patients taking anticoagulant and antiplatelet therapies have a higher chance of developing a hematoma, resulting in clinicians looking for solutions.

As briefly discussed above, the collagen used in conventional anchorage devices is a heavily processed version of collagen that has undergone milling, acid treatment, and has been pepsinized. The processing steps enable collagen to be readily solubilized and cast into films. Adversely, it also results in a reduction of molecular weight and potentially denatures the protein structure. In particular this processing can affect collagen's triple helical peptides, which are a native sequence that binds to receptors on platelets helping stimulate platelet aggregation. Smethurst, P.A., Onley, D.J., Jarvis, G.E., et al. Structural basis for the platelet-collagen interaction. Journal of Biological Chemistry, 2006; and Meyer, M. Processing of collagen based biomaterials and the resulting materials properties. Biomedical Engineering Online, 2019.

There are numerous biopolymers available that have similar film forming and solubility properties as processed collagen, while simultaneously having the potential to provide some hemostatic benefit. One example is sodium alginate. Rembre, J., Bohm, J.K., Fromm-Dornieden, C., et al. Comparison of hemostatic dressings for superficial wounds using a new spectrophotometric coagulation assay. Journal of Translational Medicine, 2015; and Timmons, J. Alginates as haemostatic agents: worth revisiting? Wounds, 2009. Across the literature, alginates have been used as hydrogels, vehicles of drug delivery, and scaffolds for wound regeneration. Yong Lee, K., Mooney, D.J. Alginate: properties and biomedical applications. Progress in Polymer Science, 2013. Alginate wound dressings have been successfully commercially marketed under brand names including AlgiSite (Smith and Nephew), Biatain Alginate Ag (Coloplast) and Tegaderm Alginate Ag Silver Dressing (3M). These products have demonstrated that alginate has a satisfactory biocompatibility and a documented hemostatic effect.

In view of the foregoing, this disclosure is directed to a surgical device that is based on sodium or calcium alginate in lieu of collagen. Alginate containing films in vitro showed an ability to clot blood quicker than collagen containing tranexamic acid films. As such, the surgical device of the present disclosure utilizes an alginate-based film containing tranexamic acid with a TYRX envelope in order to help reduce the bleeding. In some embodiments, alternative modalities can help enhance hemostatic activity or help plasticize the film coatings to make it softer and less rigid. This includes incorporation of glycerol, short chain carbohydrates such as sorbitol, mannitol, or incorporation of carboxymethylcellulose. Other plasticizers that are Generally Recognized as Safe "Gras" such as triethyl citrate, citrate esters, or propylene glycol.

The surgical device of the present disclosure has been evaluated with in-vitro blood models. At least 20% reduction in clotting time was observed when compared to collagen based films under different blood anticoagulation conditions. In particular, the surgical device of the present disclosure in vitro showed an ability to clot blood quicker than collagen containing tranexamic acid films.

Reference will now be made in detail to certain embodiments of the invention, examples of which are illustrated in the accompanying drawings. While the invention will be described in conjunction with the illustrated embodiments, it will be understood that they are not intended to limit the invention to those embodiments.

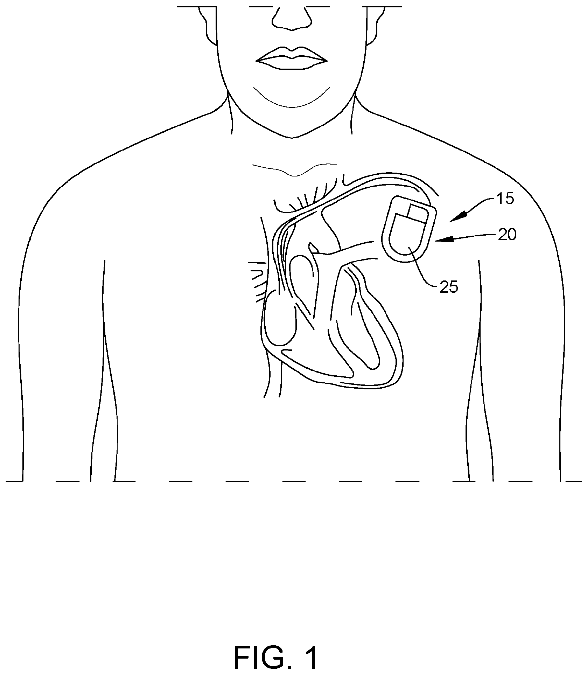

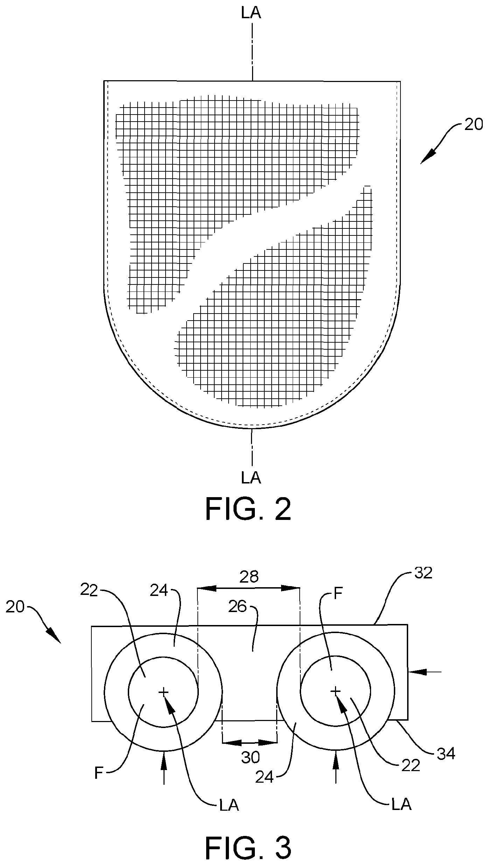

This disclosure is directed to a surgical system 15. In some embodiments, system 15 includes one or more anchorage devices, such as, for example, an anchorage device 20, as shown in FIGS. 1 and 2. In some embodiments, the components of anchorage device 20 can be fabricated from biologically acceptable materials suitable for medical applications, including metals, synthetic polymers, allografts, xenografts, isografts, ceramics and bone material and/or their composites, depending on the particular application and/or preference of a medical practitioner. For example, the components of anchorage device 20, individually or collectively, can be fabricated from materials such as stainless steel alloys, commercially pure titanium, titanium alloys, Grade 5 titanium, superelastic titanium alloys, cobalt-chrome alloys, stainless steel alloys, superelastic metallic alloys (e.g., Nitinol, super elasto-plastic metals, such as GUM METAL^{®} manufactured by Toyota Material Incorporated of Japan), ceramics and composites thereof such as calcium phosphate (e.g., SKELITE^{™} manufactured by Biologix Inc.), thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, polyethylene terephthalate (PET), fabric, silicone, polyurethane, silicone-polyurethane copolymers, polymeric rubbers, polyolefin rubbers, hydrogels, semi-rigid and rigid materials, elastomers, rubbers, thermoplastic elastomers, thermoset elastomers, elastomeric composites, rigid polymers including polyphenylene, polyamide, polyimide, polyetherimide, polyethylene, epoxy, tyrosine polyarylate, bone material including autograft, allograft, xenograft or transgenic cortical and/or corticocancellous bone, and tissue growth or differentiation factors, partially resorbable materials, such as, for example, composites of metals and calcium-based ceramics, composites of PEEK and calcium based ceramics, composites of PEEK with resorbable polymers, totally resorbable materials, such as, for example, calcium based ceramics such as calcium phosphate, tri-calcium phosphate (TCP), hydroxyapatite (HA)-TCP, calcium sulfate, or other resorbable polymers such as polylactide, polyglycolide, polytyrosine carbonate, polycaroplactone and their combinations.

Various components of anchorage device 20 may have material composites, including the above materials, to achieve various desired characteristics such as strength, rigidity, elasticity, compliance, biomechanical performance, durability and radiolucency or imaging preference. The components of anchorage device 20, individually or collectively, may also be fabricated from a heterogeneous material such as a combination of two or more of the above-described materials. The components of anchorage device 20 may be monolithically formed, integrally connected or include fastening elements and/or instruments, as described herein.

Anchorage device 20 is configured to be coupled to and/or applied to a device, such as, for example, a medical device 25, as shown in FIG. 1. In some embodiments, medical device 25 is an implantable medical device, as discussed herein. In some embodiments, medical device 25 is a non-implantable medical device, as discussed herein. In some embodiments, anchorage device 20 is configured to surround and/or enclose at least a portion of medical device 25, as discussed herein. Anchorage device 20 is configured to be secured to tissue to support one or more devices 25, such as grafts (e.g., abdominal aortic aneurysm grafts, etc.), stents, catheters (including arterial, intravenous, blood pressure, stent graft, etc.), valves (e.g., polymeric or carbon mechanical valves), embolic protection filters (including distal protection devices), vena cava filters, aneurysm exclusion devices, artificial hearts, cardiac jackets, and heart assist devices (including left ventricle assist devices), implantable defibrillators, subcutaneous implantable defibrillators, implantable monitors, for example, implantable cardiac monitors, electrostimulation devices and leads (including pacemakers, lead adapters and lead connectors), implanted medical device power supplies, peripheral cardiovascular devices, atrial septal defect closures, left atrial appendage filters, valve annuloplasty devices, mitral valve repair devices, vascular intervention devices, ventricular assist pumps, and vascular access devices (including parenteral feeding catheters, vascular access ports, central venous access catheters).

Implantable medical devices may also include, for example, surgical devices such as sutures of all types, anastomosis devices (including anastomotic closures), suture anchors, hemostatic barriers, screws, plates, clips, vascular implants, tissue scaffolds, cerebro-spinal fluid shunts, shunts for hydrocephalus, drainage tubes, catheters including thoracic cavity suction drainage catheters, abscess drainage catheters, biliary drainage products, and implantable pumps. Implantable medical devices may also include, for example, orthopedic devices such as joint implants, acetabular cups, patellar buttons, bone repair/augmentation devices, spinal devices (e.g., vertebral disks and the like), bone pins, cartilage repair devices, and artificial tendons. Implantable medical devices may also include, for example, dental devices such as dental implants and dental fracture repair devices. Implantable medical devices may also include, for example, drug delivery devices such as drug delivery pumps, implanted drug infusion tubes, drug infusion catheters, and intravitreal drug delivery devices. Implantable medical devices may also include, for example, ophthalmic devices such as scleral buckles and sponges, glaucoma drain shunts and intraocular lenses.

Implantable medical devices may also include, for example, urological devices such as penile devices (e.g., impotence implants), sphincter, urethral, prostate, and bladder devices (e.g., incontinence devices, benign prostate hyperplasia management devices, prostate cancer implants, etc.), urinary catheters including indwelling ("Foley") and non-indwelling urinary catheters, and renal devices. Implantable medical devices may also include, for example, synthetic prostheses such as breast prostheses and artificial organs (e.g., pancreas, liver, lungs, heart, etc.). Implantable medical devices may also include, for example, respiratory devices including lung catheters. Implantable medical devices may also include, for example, neurological devices such as neurostimulators, neurological catheters, neurovascular balloon catheters, neuro-aneurysm treatment coils, and neuropatches, splints, ear wicks, ear drainage tubes, tympanostomy vent tubes, otological strips, laryngectomy tubes, esophageal tubes, esophageal stents, laryngeal stents, salivary bypass tubes, and tracheostomy tubes. Implantable medical devices may also include, for example, oncological implants. Implantable medical devices may also include, for example, pain management implants.

In some embodiments, anchorage device 20 is configured to be coupled to and/or applied to or to surround and/or enclose at least a portion of a non-implantable medical device, as discussed herein. Non-implantable devices can include dialysis devices and associated tubing, catheters, membranes, and grafts; autotransfusion devices; vascular and surgical devices including atherectomy catheters, angiographic catheters, intraaortic balloon pumps, intracardiac suction devices, blood pumps, blood oxygenator devices (including tubing and membranes), blood filters, blood temperature monitors, hemoperfusion units, plasmapheresis units, transition sheaths, dialators, intrauterine pressure devices, clot extraction catheters, percutaneous transluminal angioplasty catheters, electrophysiology catheters, breathing circuit connectors, stylets (vascular and non-vascular), coronary guide wires, peripheral guide wires; dialators (e.g., urinary, etc.); surgical instruments (e.g. scalpels and the like); endoscopic devices (such as endoscopic surgical tissue extractors, esophageal stethoscopes); and general medical and medically related devices including blood storage bags, umbilical tape, membranes, gloves, surgical drapes, wound dressings, wound management devices, needles, percutaneous closure devices, transducer protectors, pessary, uterine bleeding patches, PAP brushes, clamps (including bulldog clamps), cannulae, cell culture devices, materials for in vitro diagnostics, chromatographic support materials, infection control devices, colostomy bag attachment devices, birth control devices; disposable temperature probes; and pledgets.

Anchorage device 20 can have a variety of different configurations, shapes and sizes. For example, anchorage device 20 can be provided with a size and shape or other configuration that can provide the functionality of supporting and immobilizing medical device 25 at a treatment site within a patient's body, while also improving the removability of anchorage device 20 after the treatment has been completed. In some embodiments, medical device 25 can be disposed within a pocket defined by anchorage device 20 and anchorage device 20 can be implanted and secured to tissue at a desired treatment site within a body of a patient. As discussed herein, during implantation, scar tissue can form at the treatment site and/or tissue can become ingrown within anchorage device 20. After the treatment is completed, medical device 25 can remain in the patient as discussed below or can be removed from the patient leaving anchorage device 20 implanted. To remove anchorage device 20, tissue that is ingrown within anchorage device 20 can be cut or otherwise detached from anchorage device 20. In some embodiments, a portion of anchorage device 20 may not be removable from the tissue and will remain implanted within the patient.

In some embodiments, anchorage device 20 includes a substrate, such as, for example, substrate 22, as shown in FIGS. 3, 6 and 10, for example. In some embodiments, substrate 22 acts as a foundation or skeleton upon which one or more coatings may be applied that at least partially coat substrate 22, for example. In some embodiments, substrate 22 is provided in the form of a mesh. In some embodiments, the mesh is a web or fabric with a construction of knitted, braided, woven or non-woven filaments or fibers that are interlocked in such a way to create a fabric or a fabric-like material that includes a matrix of filaments that define multiple pores or apertures, such as, for example, apertures 28, as discussed herein. That is, the space between adjacent filaments or fibers define pores or apertures of the mesh, such as, for example, apertures 28, as discussed herein. In some embodiments, the pores or apertures may be beneficial to allow tissue in-growth, for example.

In some embodiments, substrate 22 may be formed with one or more biocompatible materials, which may be synthetic or naturally occurring. In some embodiments, the one or more biocompatible materials include, for example, polypropylene, polyester, polytetrafluoroethylene, polyamides, silicones, polysulfones, metals, alloys, titanium, stainless steel, shape memory metals (e.g. Nitinol), and/or combinations thereof.

In some embodiments, anchorage device 20 is configured to be implanted temporarily within a body of a patient and/or is configured to be removed (e.g., explanted) from the patient's body after a period of time. In such embodiments, substrate 22 may include a non-biodegradable material and/or a non-bioresorbable material. For example, substrate 22 may be made entirely from a non-biodegradable material and/or a non-bioresorbable material such that substrate 22 is made only from the non-biodegradable material and/or non-bioresorbable material. In some embodiments, substrate 22 may include one or more non-biodegradable and/or a non-bioresorbable material and one or more biodegradable and/or resorbable material. In some embodiments, one side of substrate 22 may include one or more non-biodegradable and/or a non-bioresorbable material and another side of substrate 22 can include one or more biodegradable and/or resorbable material.

As used herein, the term "biodegradable" refers to, for example, a material that can be at least partially broken down or degraded by a bodily fluid and discarded as waste from the body and/or a material that can be broken down or degraded by a living organism. Thus, "non-biodegradable" can refer to a material that cannot be broken down or degraded by a bodily fluid and/or cannot be broken down or degraded by a living organism. As used herein the term "resorbable" refers to, for example, a material that can be at least partially broken down or degraded by a bodily fluid and assimilated within the body. Thus, a "non-resorbable" material as used herein can refer to, for example, a material that cannot be broken down or degraded by bodily fluid and assimilated within the body.

In some embodiments, the biocompatible biodegradable and/or bioresorbable material or materials may include polymeric and/or non-polymeric materials, such as, for example, one or more poly (alpha-hydroxy acids), poly (lactide-co-glycolide) (PLGA), polylactide (PLA), poly(L-lactide), polyglycolide (PG), polyethylene glycol (PEG) conjugates of poly (alpha-hydroxy acids), polyorthoesters (POE), polyaspirins, polyphosphazenes, collagen, hydrolyzed collagen, gelatin, hydrolyzed gelatin, fractions of hydrolyzed gelatin, elastin, starch, pre-gelatinized starch, hyaluronic acid, chitosan, alginate, albumin, fibrin, vitamin E analogs, such as alpha tocopheryl acetate, d-alpha tocopheryl succinate, D,L-lactide, or L-lactide, -caprolactone, dextrans, vinylpyrrolidone, polyvinyl alcohol (PVA), PVA-g-PLGA, PEGT-PBT copolymer (polyactive), methacrylates, poly (N-isopropylacrylamide), PEO-PPO-PEO (pluronics), PEO-PPO-PAA copolymers, PLGA-PEO-PLGA, PEG-PLG, PLA-PLGA, poloxamer 407, PEG-PLGA-PEG triblock copolymers, POE, SAIB (sucrose acetate isobutyrate), polydioxanone, methylmethacrylate (MMA), MMA and N-vinylpyyrolidone, polyamide, oxycellulose, copolymer of glycolic acid and trimethylene carbonate, polyesteramides, tyrosine polyarylates, polyetheretherketone, polymethylmethacrylate, silicone, hyaluronic acid, chitosan, or combinations thereof. In one embodiment, substrate 22 comprises Glycoprene, which is sold by Poly-Med, Inc. As used herein, the term "glycoprene" or "Glycoprene" refers to Glycoprene^{®} or Glycoprene II^{®}. Glycoprene^{®} can refer to different variations of the material sold under the trade name Glycoprene^{®}, such as, for example, Glycoprene^{®}6829, Glycoprene^{®}8609 and Glycoprene^{®}7027.

In some embodiments, the biocompatible non-biodegradable and/or non-bioresorbable material or materials may include polymeric and/or non-polymeric materials, such as, for example, polyurethane, polyester, polytetrafluoroethylene (PTFE), polyethylacrylate/polymethylmethacrylate, polylactide, polylactide-co-glycolide, polyamides, polydioxanone, polyvinyl chloride, polymeric or silicone rubber, collagen, thermoplastics, or combinations thereof.

In some embodiments, anchorage device 20 is configured to be permanently implanted within a body of a patient. In such embodiments, substrate 22 may include a biodegradable material and/or a bioresorbable material. For example, substrate 22 may be made entirely from a biodegradable material and/or a bioresorbable material such that substrate 22 is made only from the biodegradable material and/or bioresorbable material.

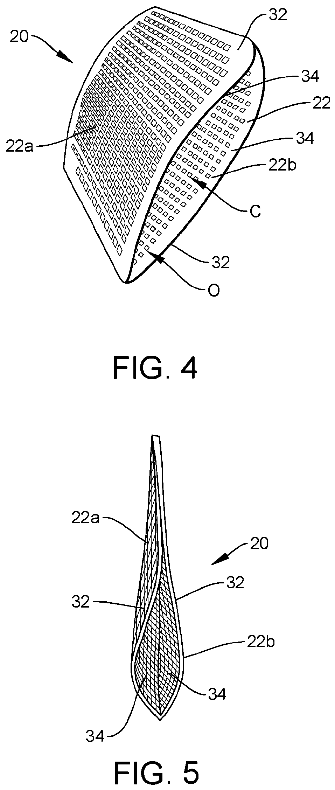

In some embodiments, substrate 22 is provided in the form of a sheet. In some embodiments, substrate 22 includes only one sheet, such as, for example, sheet 22a, and in other embodiments, substrate 22 includes at least one sheet, such as, for example, sheet 22b, in addition to sheet 22a, as shown in FIGS. 4 and 5 for example. In some embodiments, sheet 22b is separate from sheet 22a and/or is removably attached to sheet 22a. In some embodiments, sheet 22b is joined with sheet 22a to form a structure configured to at least partially surround medical device 25, as discussed herein. In some embodiments, sheet 22a is identical to sheet 22b. In some embodiments, sheet 22a is different than sheet 22b.

In some embodiments, anchorage device 20 includes a coating, such as, for example, a first coating 24 that is applied directly to substrate 22 such that coating 24 covers all or a portion of substrate 22. Coating 24 includes a polymer and an API dispersed in the polymer such that the polymer releases the API as the polymer degrades. According to present claim 1, the coating 24 includes antibiotics.

The API of coating 24 can include one or more antibiotics, such as, for example, rifampin and minocycline. In some embodiments, the API of coating 24 can include a mixture of rifampin and minocycline. In some embodiments, the API of coating 24 can include any of the APIs discussed herein in place of, or in addition to, a mixture of rifampin and minocycline. In some embodiments, the polymer of coating 24 can include one or more of the polymers discussed herein. In some embodiments, the polymer of coating 24 can include a tyrosine-derived polyarylate, such as, for example, one or more of the tyrosine-derived polyarylates discussed herein.

In some embodiments, anchorage device 20 includes a coating, such as, for example, second coating 26 that is applied directly to coating 24 such that coating 26 covers all or a portion of coating 24. According to present claim 1, the coating 26 includes a polymer and a hemostatic agent dispersed in the polymer of coating 26 such that the polymer of coating 26 releases the API of coating 26 as the polymer of coating 26 degrades. In some embodiments, the API of coating 26 can include one or more of the hemostatic agents discussed herein. In some embodiments, the hemostatic agent is tranexamic acid. The polymer of coating 26 can include one or more of the polymers discussed herein. According to present claim 1, the polymer of coating 26 includes an alginate. In some embodiments, the polymer of coating 26 can include one or more alginates selected from the group consisting of calcium alginate and sodium alginate, as discussed herein. In some embodiments, the alginate of coating is Medium viscosity Sodium Alginate: 1000-2000 cps sodium alginate. It is noted that providing a coating that is made from a hemostatic agent, such as, for example, one or more alginates that is configured to release one or more other hemostatic agents, such as, for example, tranexamic acid and/or other hemostatic agent(s) provides a dual hemostatic effect designed to achieve hemostasis faster than devices that use only one type of hemostatic agent and hence provide only a single hemostatic effect.

In some embodiments, coating 24 is applied to substrate 22 such that coating 24 directly engages fibers, such as, for example, fibers F that form substrate 22 such that coating 24 extends 360 degrees about each of fibers F along at least a portion of a length of each of fibers F, as shown in FIG. 3, for example. That is, fibers F each have a length extending along a longitudinal axis LA and coating 24 extends 360 degrees about axis LA of each fiber F. In some embodiments, coating 24 extends 360 degrees about each of fibers F along the entire length of each of fibers F. In some embodiments, coating 24 has a uniform thickness 360 degrees about each of fibers F along a length of each of fibers F.

In some embodiments, coating 26 is spaced apart from substrate 22 by coating 24, as shown in FIG. 3, for example. In some embodiments, coating 26 directly engages coating 24 such that coating 26 extends less than 360 degrees about each of fibers F along at least a portion of the length of each of fibers F. That is, coating 26 extends less 360 than degrees about axis LA of each fiber F. In some embodiments, coating 26 extends only between about 150 degrees and about 330 degrees about axis LA of each fiber F. In some embodiments, coating 26 extends only between about 170 degrees and about 310 degrees about axis LA of each fiber F. In some embodiments, coating 26 extends only between about 190 degrees and about 290 degrees about axis LA of each fiber F. In some embodiments, coating 26 extends only between about 210 degrees and about 270 degrees about axis LA of each fiber F. In some embodiments, coating 26 extends only between about 230 degrees and about 250 degrees about axis LA of each fiber F. In some embodiments, coating 26 extends only about 240 degrees about axis LA of each fiber F. This configuration provides device 20 with a surface 32 and an opposite surface 34. In some embodiments, coating 26 solely defines surface 32 and coatings 24, 26 define surface 34, as shown in FIG. 3, for example. The configuration of surface 32 and surface 34 allows device 20 to be selectively implanted in a patient such that device 20 releases a hemostatic agent, such as, for example, tranexamic acid to be released from coating 26 before the antibiotic(s), such as, for example, rifampin and/or minocycline and/or other API(s) is/are released from coating 24. For example, positioning device 20 within a patient such that surface 32 is exposed to fluid or surface 32 is exposed to more fluid than surface 34 will cause coating 26 to degrade before coating 24 degrades such that the tranexamic acid and/or other hemostatic agent(s) is released from coating 26 before rifampin and/or minocycline and/or other API(s) is/are released from coating 24.

In some embodiments, wherein device 20 is in the form of a pocket or pouch having piece 22a joined with piece 22b as discussed herein, surface 34 of piece 22a faces surface 34 of piece 22b such that surfaces 34, 34 of pieces 22a, 22b define cavity C. This configuration may be desirable to allow coating 26 to come into contact with fluid upon implantation before coating 24 comes into contact with fluid to allow coating 26 to release tranexamic acid and/or other hemostatic agent(s) to promote blood clotting before coating 24 releases the antibiotics and/or other API(s) to prevent and/or treat infection.

In some embodiments, wherein device 20 is in the form of a pocket or pouch having piece 22a joined with piece 22b as discussed herein, surface 32 of piece 22a faces surface 32 of piece 22b such that surfaces 32, 32 of pieces 22a, 22b define cavity C. This configuration may be desirable to allow coating 24 and coating 26 to come into contact with fluid simultaneously upon implantation to allow coating 26 to release tranexamic acid and/or other hemostatic agent(s) to promote blood clotting at the same time or at approximately the same time as coating 24 releases the antibiotics and/or other API(s) to prevent and/or treat infection. In some embodiments, the polymers of coatings 24, 26 can be customized with regard to the timing in which the polymers release the antibiotics and/or other API(s) and tranexamic acid and/or other hemostatic agent(s). For example, in some embodiments, the polymers may be customized such that the polymer of coating 26 releases the tranexamic acid and/or other hemostatic agent(s) before the polymer of coating 24 releases the antibiotics and/or other API(s) when coatings 24, 26 are exposed to fluid simultaneously. In some embodiments, the polymers may be customized such that the polymer of coating 26 releases the tranexamic acid and/or other hemostatic agent(s) after the polymer of coating 24 releases the antibiotics and/or other API(s) when coatings 26 are exposed to fluid simultaneously. In some embodiments, the polymers may be customized such that the polymer of coating 26 releases the tranexamic acid and/or other hemostatic agent(s) at the same time that the polymer of coating 24 releases the antibiotics and/or other API(s) when coatings 26 are exposed to fluid simultaneously.

In some embodiments, the duration of release of the active agents in coatings 24, 26 can be at least somewhat controlled by the polymers that are included in coating 24 and coating 26. For example, the polymer used in coating 26 can be configured to degrade at a faster rate than the polymer used in coating 24 such that tranexamic acid and/or other hemostatic agent(s) is released from coating 26 before rifampin and/or minocycline and/or other API(s) is/are released from coating 24, even if surfaces 32, 34 are both exposed to fluid, including the same amount of fluid.

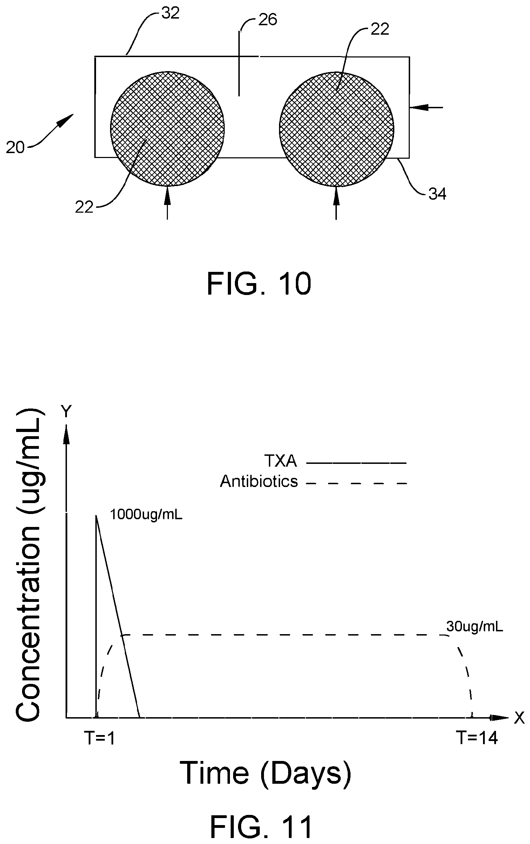

In that coating 26 can release tranexamic acid and/or other hemostatic agent(s) to promote blood clotting, it may be desirable to release the tranexamic acid and/or other hemostatic agent(s) from coating 26 as soon as possible following the implantation of device 20. This will allow the tranexamic acid and/or other hemostatic agent(s) to act before the polymer of coating 24 degrades and releases rifampin and/or minocycline and/or other API(s). As such, in some embodiments, it may be desirable to provide coating 26 with a polymer that degrades over a few hours to a few days and to provide coating 24 with a polymer that degrades over multiple days or weeks. This will allow device 20 to release a substantial amount of tranexam ic acid and/or other hemostatic agent(s) to initiate healing soon after implantation of device 20 and to release rifampin and/or minocycline and/or other API(s) during and after such healing to prevent infection, as shown in FIG. 11. In some embodiments, coating 26 includes a polymer that degrades over about one or two hours to about one or two days and coating 24 includes a polymer that degrades over about nine to twelve weeks. In some embodiments, coating 26 includes a polymer that degrades over one or two hours and coating 24 includes a polymer that degrades over about nine weeks. In some embodiments, coating 26 includes a polymer that degrades over one or two days and coating 24 includes a polymer that degrades over about nine to twelve weeks.

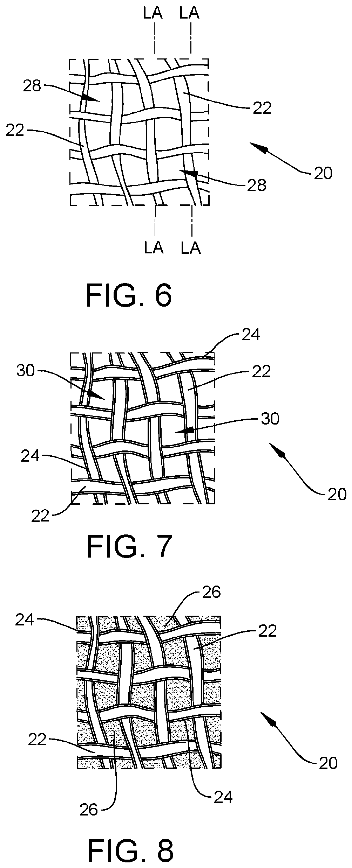

In some embodiments, coating 24 is applied to substrate 22 such that coating 24 at least partially coats filaments or fibers of substrate 22, as shown in FIGS. 3, 7 and 10, without filling apertures 28 of substrate 22, as shown in FIG. 6. That is, coating 24 has a thickness that defines gaps 30 between adjacent filaments or fibers of substrate 22, as shown in FIG. 7. In some embodiments, coating is applied to substrate 22 such that coating 26 fills gaps 30, as shown in FIG. 8. That is, coating 26 covers or fills gaps 30 and coating 26 and coating 24 cover or fill apertures 28 when device 20 is implanted and then exposes gaps 30 as coating 26 degrades. That is, gaps 30 become uncovered and/or unfilled post-implantation as coating 26 degrades. Apertures 28 are then exposed as coating 24 degrades. That is, apertures 28 become uncovered or unfilled as coating 24 degrades.

In some embodiments, substrate 22 is biodegradable and/or bioresorbable and device 20 is configured to hold medical device 25 therein such that substrate 22 does not begin to degrade until coatings 24, 26 completely degrade such that device 20 can hold medical device 25 therein until all of the tranexamic acid and/or other hemostatic agent(s) and antibiotics and/or other API(s) are released from coatings 24, 26. In some embodiments, substrate 22 does not begin to degrade until coatings 24, 26 completely degrade because substrate 22 is not directly exposed to fluid until coatings 24, 26 completely degrade. In some embodiments, substrate 22 has a degradation rate that is slower than that of the polymers in coatings 24, 26 such that substrate 22 will degrade slower than coatings 24, 26 even when substrate 22 and coatings 24,26 are exposed to fluid at the same time. In some embodiments, substrate 22 is completely biodegradable or bioresorbable. That is, all of substrate 22 is biodegradable or bioresorbable. In some embodiments, substrate 22 is completely non-biodegradable and/or non-bioresorbable. That is no portion of substrate 22 is biodegradable or bioresorbable.

Coating 26 includes a hemostatic agent that can promote hemostasis, as discussed herein. One such hemostatic agent is tranexamic acid. However, it is envisioned that coating 26 can include one or more hemostatic agents in addition to or in place of tranexamic acid, such as, for example, epinephrine, collagen (disclosed, but not claimed) chitosan and oxidized regenerated cellulose. According to the present disclosure, the collagen can include acid soluble collagen, pepsin soluble collagen, gelatin, cross-linkable collagen, or fibrillar collagen. According to present claim 1, the coating 26 comprises at least a hemostatic agent. In some embodiments, coating 26 can include one or more hemostatic agents in addition to or in place of tranexam ic acid, such as, for example, one or more of Spongostan^{®}, Surgifoam^{®}, Avitene, thrombin and Ostene^{®} in addition to or in place of the hemostatic agents discussed above. In some embodiments, coating 26 can include one or more hemostatic agents in addition to or in place of tranexamic acid, such as, for example, one or more of protam ine, norepinephrine, desmopressin, lysine analogs, gelatin, polysaccharide spheres, mineral zeolite, bovine thrombin, pooled human thrombin, recombinant thrombin, gelatin and thrombin, collagen and thrombin, cyanacrylate, fibrin glue, polyethylene glycol, and glutaraldehyde in addition to or in place of the hemostatic agents discussed above. In some embodiments, coating 26 can include one or more hemostatic agents in addition to or in place of tranexamic acid, such as, for example, a mixture or combination of the hemostatic agents discussed herein. In some embodiments, the lysine analog is tranexamic acid.

In some embodiments, the API of coating 24 can include one or a combination of active pharmaceutical ingredients, such as, for example, anesthetics, antibiotics, anti-inflammatory agents, procoagulant agents, fibrosis-inhibiting agents, anti-scarring agents, antiseptics, leukotriene inhibitors/antagonists, cell growth inhibitors and mixtures thereof. According to present claim 1, the API of coating 24 includes one or more antibiotic. In some embodiments, the antibiotic is selected from the group consisting of rifampin and minocycline and mixtures thereof.

Examples of non-steroidal anti-inflammatories include, but are not limited to, naproxen, ketoprofen, ibuprofen as well as diclofenac; celecoxib; sulindac, diflunisal; piroxicam; indomethacin; etodolac; meloxicam; r-flurbiprofen; mefenamic; nabumetone; tolmetin, and sodium salts of each of the foregoing; ketorolac bromethamine; ketorolac bromethamine tromethamine; choline magnesium trisalicylate; rofecoxib; valdecoxib; lumiracoxib; etoricoxib; aspirin; salicylic acid and its sodium salt; salicylate esters of alpha, beta, gamma-tocopherols and tocotrienols (and all their d, 1, and racemic isomers); and the methyl, ethyl, propyl, isopropyl, n-butyl, sec-butyl, t-butyl, esters of acetylsalicylic acid.

Examples of anesthetics include, but are not limited to, licodaine, bupivacaine, and mepivacaine. Further examples of analgesics, anesthetics and narcotics include, but are not limited to acetaminophen, clonidine, benzodiazepine, the benzodiazepine antagonist flumazenil, lidocaine, tramadol, carbamazepine, meperidine, zaleplon, trimipramine maleate, buprenorphine, nalbuphine, pentazocain, fentanyl, propoxyphene, hydromorphone, methadone, morphine, levorphanol, and hydrocodone. Local anesthetics have weak antibacterial properties and can play a dual role in the prevention of acute pain and infection.

Examples of antibacterial agents or antimicrobials include, but are not limited to, triclosan, chlorohexidine and other cationic biguanides, rifampin, minocycline (or other tetracycline derivatives), vancomycin, gentamycin; gendine; genlenol; genfoctol; clofoctol; cephalosporins and the like. Further antibacterial agents or antimicrobials include aztreonam; cefotetan and its disodium salt; loracarbef; cefoxitin and its sodium salt; cefazolin and its sodium salt; cefaclor; ceftibuten and its sodium salt; ceftizoxime; ceftizoxime sodium salt; cefoperazone and its sodium salt; cefuroxime and its sodium salt; cefuroxime axetil; cefprozil; ceftazidime; cefotaxime and its sodium salt; cefadroxil; ceftazidime and its sodium salt; cephalexin; hexachlorophene; cefamandole nafate; cefepime and its hydrochloride, sulfate, and phosphate salt; cefdinir and its sodium salt; ceftriaxone and its sodium salt; cefixime and its sodium salt; cetylpyridinium chloride; ofoxacin; linexolid; temafloxacin; fleroxacin; enoxacin; gemifloxacin; lomefloxacin; astreonam; tosufloxacin; clinafloxacin; cefpodoxime proxetil; chloroxylenol; methylene chloride, iodine and iodophores (povidone-iodine); nitrofurazone; meropenem and its sodium salt; imipenem and its sodium salt; cilastatin and its sodium salt; azithromycin; clarithromycin; dirithromycin; erythromycin and hydrochloride, sulfate, or phosphate salts ethylsuccinate, and stearate forms thereof, clindamycin; clindamycin hydrochloride, sulfate, or phosphate salt; lincomycin and hydrochloride, sulfate, or phosphate salt thereof, tobramycin and its hydrochloride, sulfate, or phosphate salt; streptomycin and its hydrochloride, sulfate, or phosphate salt; vancomycin and its hydrochloride, sulfate, or phosphate salt; neomycin and its hydrochloride, sulfate, or phosphate salt; acetyl sulfisoxazole; colistimethate and its sodium salt; quinupristin; dalfopristin; amoxicillin; ampicillin and its sodium salt; clavulanic acid and its sodium or potassium salt; penicillin G; penicillin G benzathine, or procaine salt; penicillin G sodium or potassium salt; carbenicillin and its disodium or indanyl disodium salt; piperacillin and its sodium salt; α-terpineol; thymol; taurinamides; nitrofurantoin; silver-sulfadiazine; hexetidine; methenamine; aldehydes; azylic acid; silver; benzyl peroxide; alcohols; carboxylic acids; salts; nafcillin; ticarcillin and its disodium salt; sulbactam and its sodium salt; methylisothiazolone, moxifloxacin; amifloxacin; pefloxacin; nystatin; carbepenems; lipoic acids and its derivatives; beta-lactams antibiotics; monobactams; aminoglycosides; microlides; lincosamides; glycopeptides; tetracyclines; chloramphenicol; quinolones; fucidines; sulfonamides; macrolides; ciprofloxacin; ofloxacin; levofloxacins; teicoplanin; mupirocin; norfloxacin; sparfloxacin; ketolides; polyenes; azoles; penicillins; echinocandines; nalidixic acid; rifamycins; oxalines; streptogramins; lipopeptides; gatifloxacin; trovafloxacin mesylate; alatrofloxacin mesylate; trimethoprims; sulfamethoxazole; demeclocycline and its hydrochloride, sulfate, or phosphate salt; doxycycline and its hydrochloride, sulfate, or phosphate salt; minocycline and its hydrochloride, sulfate, or phosphate salt; tetracycline and its hydrochloride, sulfate, or phosphate salt; oxytetracycline and its hydrochloride, sulfate, or phosphate salt; chlortetracycline and its hydrochloride, sulfate, or phosphate salt; metronidazole; dapsone; atovaquone; rifabutin; linezolide; polymyxin B and its hydrochloride, sulfate, or phosphate salt; sulfacetamide and its sodium salt; and clarithromycin (and combinations thereof). In some embodiments the polymer may contain rifampin and another antimicrobial agent, such as, for example, an antimicrobial agent that is a tetracycline derivative. In some embodiments, the polymer contains a cephalosporin and another antimicrobial agent. In some embodiments, the polymer contains combinations including rifampin and minocycline, rifampin and gentamycin, and rifampin and minocycline.

When a mixture of two antibiotics is used, they generally present in a ratio ranging from about 10:1 to about 1:10. In some embodiments, a mixture of rifampin and minocycline are used. In those embodiments, a ratio of rifampin to minocycline ranges from about 5:2 to about 2:5. In other embodiments, the ratio of rifampin to minocycline is about 1:1.

Examples of antifungals include amphotericin B; pyrimethamine; flucytosine; caspofungin acetate; fluconazole; griseofulvin; terbinafine and its hydrochloride, sulfate, or phosphate salt; amorolfine; triazoles (Voriconazole); flutrimazole; cilofungin; LY303366 (echinocandines); pneumocandin; imidazoles; omoconazole; terconazole; fluconazole; amphotericin B, nystatin, natamycin, liposomal amptericin B, liposomal nystatins; griseofulvin; BF-796; MTCH 24; BTG-137586; RMP-7/Amphotericin B; pradimicins; benanomicin; ambisome; ABLC; ABCD; Nikkomycin Z; flucytosine; SCH 56592; ER30346; UK 9746; UK 9751; T 8581; LY121019; ketoconazole; micronazole; clotrimazole; econazole; ciclopirox; naftifine; and itraconazole.

In some embodiments, active pharmaceutical ingredient (API) of coating 24 includes keflex, acyclovir, cephradine, malphalen, procaine, ephedrine, adriamycin, daunomycin, plumbagin, atropine, quinine, digoxin, quinidine, biologically active peptides, cephradine, cephalothin, cis-hydroxy-L-proline, melphalan, penicillin V, aspirin, nicotinic acid, chemodeoxycholic acid, chlorambucil, paclitaxel, sirolimus, cyclosporins, 5-fluorouracil and the like.

In some embodiments, the API of coating 24 includes one or more ingredients that act as angiogenensis inhibitors or inhibit cell growth such as epidermal growth factor, PDGF, VEGF, FGF (fibroblast growth factor) and the like. These ingredients include anti-growth factor antibodies (neutrophilin-1), growth factor receptor-specific inhibitors such as endostatin and thalidomide. Examples of useful proteins include cell growth inhibitors such as epidermal growth factor.

Examples of anti-inflammatory compounds include, but are not limited to, anecortive acetate; tetrahydrocortisol, 4,9(11)-pregnadien-17α, 21-diol-3,20-dione and its -21-acetate salt; 111-epicortisol; 17α-hydroxyprogesterone; tetrahydrocortexolone; cortisona; cortisone acetate; hydrocortisone; hydrocortisone acetate; fludrocortisone; fludrocortisone acetate; fludrocortisone phosphate; prednisone; prednisolone; prednisolone sodium phosphate; methylprednisolone; methylprednisolone acetate; methylprednisolone, sodium succinate; triamcinolone; triamcinolone-16,21-diacetate; triamcinolone acetonide and its -21-acetate, -21-disodium phosphate, and -21-hemisuccinate forms; triamcinolone benetonide; triamcinolone hexacetonide; fluocinolone and fluocinolone acetate; dexamethasone and its -21-acetate, -21-(3,3-dimethylbutyrate), -21-phosphate disodium salt, -21-diethylaminoacetate, -21-isonicotinate, -21-dipropionate, and -21-palmitate forms; betamethasone and its -21-acetate, -21-adamantoate, -17-benzoate, -17,21-dipropionate, -17-valerate, and -21-phosphate disodium salts; beclomethasone; beclomethasone dipropionate; diflorasone; diflorasone diacetate; mometasone furoate; and acetazolamide.

Examples of leukotriene inhibitors/antagonists include, but are not limited to, leukotriene receptor antagonists such as acitazanolast, iralukast, montelukast, pranlukast, verlukast, zafirlukast, and zileuton.

In some embodiments, active pharmaceutical ingredient (API) of coating 24 includes sodium 2-mercaptoethane sulfonate ("MESNA"). MESNA has been shown to diminish myofibroblast formation in animal studies of capsular contracture with breast implants [Ajmal et al. (2003) Plast. Reconstr. Surg. 112:1455-1461] and may thus act as an anti-fibrosis agent.

Procoagulants include, but are not limited to, zeolites, thrombin, and coagulation factor concentrates.

In some embodiments, the amount of the API of coating 24 that is applied to substrate 22 via coating 24 or otherwise ranges between about 0.3 to about 2.8 micrograms/cm². In other embodiments, the amount of the API of coating 24 that is applied to substrate 22 via coating 24 or otherwise ranges between about 0.6 to about 1.4 micrograms/cm². In yet other embodiments, the amount of the API of coating 24 that is applied to substrate 22 via coating 24 or otherwise ranges between about 0.85 to about 1.20 micrograms/cm². In yet further embodiments, the amount of the API of coating 24 that is applied to substrate 22 via coating 24 or otherwise ranges between about 0.90 to about 1.10 micrograms/cm². In yet further embodiments, the amount of the API of coating 24 that is applied to substrate 22 via coating 24 or otherwise ranges between about 50 to about 150 micrograms/cm². In yet further embodiments, the amount of the API of coating 24 that is applied to substrate 22 via coating 24 or otherwise ranges between about 62 to about 140 micrograms/cm². In yet further embodiments, 62 micrograms/cm² of the API of coating 24 is applied to substrate 22 via coating 24 or otherwise. In yet further embodiments, 140 micrograms/cm² of the API of coating 24 is applied to substrate 22 via coating 24 or otherwise.

In some embodiments, the amount of the tranexamic acid and/or other hemostatic agent(s) that is applied to substrate 22 via coating 26 or otherwise ranges between about 0.3 to about 2.8 micrograms/cm². In other embodiments, the amount of tranexam ic acid and/or other hemostatic agent(s) that is applied to substrate 22 via coating 26 or otherwise ranges between about 0.6 to about 1.4 micrograms/cm². In yet other embodiments, the amount of tranexamic acid and/or other hemostatic agent(s) that is applied to substrate 22 via coating 26 or otherwise ranges between about 0.85 to about 1.20 micrograms/cm². In yet further embodiments, the amount of tranexamic acid and/or other hemostatic agent(s) that is applied to substrate 22 via coating 26 or otherwise ranges between about 0.90 to about 1.10 micrograms/cm². In yet further embodiments, the amount of tranexamic acid and/or other hemostatic agent(s) that is applied to substrate 22 via coating 26 or otherwise ranges between about 50 to about 150 micrograms/cm². In yet further embodiments, the amount of tranexamic acid and/or other hemostatic agent(s) that is applied to substrate 22 via coating 24 or otherwise ranges between about 62 to about 140 micrograms/cm². In yet further embodiments, 62 micrograms/cm² of tranexamic acid and/or other hemostatic agent(s) that are applied to substrate 22 via coating 26 or otherwise. In yet further embodiments, 140 micrograms/cm² of tranexamic acid and/or other hemostatic agent(s) is applied to substrate 22 via coating 26 or otherwise.

In other embodiments, the API of coating 24 includes rifampin and minocycline and the amount of each of rifampin and minocycline that is applied to substrate 22 via coating 24 ranges between about 0.6 to about 1.4 micrograms/cm². In yet other embodiments, the amount of each of rifampin and minocycline that is applied to substrate 22 via coating 24 ranges between about 0.85 to about 1.20 micrograms/cm². In yet further embodiments, the amount of each of rifampin and minocycline that is applied to substrate 22 via coating 24 ranges between about 0.90 to about 1.10 micrograms/cm².

The API of coating 24 may include one or more of the active pharmaceutical ingredients discussed herein. Doses of the APIs discussed herein are known and the amounts of any single API to include in anchorage device 20 can readily be surmised. Any pharmaceutically acceptable form of APIs discussed herein can be employed in anchorage device 20, e.g., the free base or a pharmaceutically acceptable salt or ester thereof. Pharmaceutically acceptable salts, for instance, include sulfate, lactate, acetate, stearate, hydrochloride, tartrate, maleate, citrate, phosphate and the like.

The polymer of coating 24 and/or the polymer of coating 26 may be selected from the group consisting of polylactic acid, polvalvcolic acid, polt(L-lactide), poly(D,L-lactide)polyglycolic acid[polyglycolide], poly(L-lactide-co-D,L-lactide), poly(L-lactide-co-glycolide), poly(D, L-Iadide-co-glycolide), poly(glycolide-co-trimethylene carbonate), poly(D, L-lactide-co-caprolactone), poly(glycolide-co-caprolactone), polyethylene oxide, polydioxanone, polypropylene fumarate, poly(ethyl glutamate-co-glutamic acid), poly(tert-butyloxy-carbonylmethyl glutamate), polycaprolactone, polycaprolactone co-butylacrylate, polyhydroxybutyrate, copolymers of polyhydroxybutyrate, poly(phosphazene), poly(phosphate ester), poly(amino acid), polydepsipeptides, maleic anhydride copolymers, polyiminocarbonates, poly[(97.5% dimethyl-trimethylene carbonate)-co-(2.5% trimethylene carbonate)], poly(orthoesters), tyrosine-derived polyarylates, tyrosine-derived polycarbonates, tyrosine-derived polyiminocarbonates, tyrosine-derived polyphosphonates, polyethylene oxide, polyethylene glycol, polyalkylene oxides, hydroxypropylmethylcellulose, polysaccharides such as hyaluronic acid, chitosan and regenerate cellulose. In some embodiments, the polymer of coating 24 and/or the polymer of coating 26 may include combinations, blends or mixtures of the polymers discussed herein. According to present claim 1, the coating 26 comprises an alginate.

In some embodiments, the polymer of coating 24 is a polyarylate. In some embodiments, the polymer of coating 24 and/or the polymer of coating 26 is a tyrosine-derived polyarylate. In some embodiments, the tyrosine-derived polyarylate is p(DTE co X % DT succinate), where X is about 10% to about 30%. In some embodiments, the tyrosine-derived polyarylate is p(DTE co X % DT succinate), where X ranges from about 26.5% to about 28.5%. In some embodiments, the tyrosine-derived polyarylate is p(DTE co X % DT succinate), where X is about 27.5%. In some embodiments, the polymer is P22-27.5 DT.

As used herein, DTE is the diphenol monomer desaminotyrosyl-tyrosine ethyl ester; DTBn is the diphenol monomer desaminotyrosyl-tyrosine benzyl ester; DT is the corresponding free acid form, namely desaminotyrosyl-tyrosine. BTE is the diphenol monomer 4-hydroxy benzoic acid-tyrosyl ethyl ester; BT is the corresponding free acid form, namely 4-hydroxy benzoic acid-tyrosine.

P22-XX is a polyarylate copolymer produced by condensation of DTE and DTBn with succinic acid followed by removal of benzyl group. P22-10, P22-15, P22-20, P22-XX, etc., represents copolymers different percentage of DT (i.e., 10, 15, 20 and % DT, etc.) In some embodiments, the polymer is produced by condensation of DTBn with succinic acid followed by removal of benzyl group. This polymer is represented as P02-100.

In some embodiments, the polymer of coating 24 and/or the polymer of coating 26 includes one or more polyarylates that are copolymers of desaminotyrosyl-tyrosine (DT) and an desaminotyrosyl-tyrosyl ester (DT ester), wherein the copolymer comprises from about 0.001% DT to about 80% DT and the ester moiety can be a branched or unbranched alkyl, alkylaryl, or alkylene ether group having up to 18 carbon atoms, any group of which can, optionally have a polyalkylene oxide therein. Similarly, another group of polyarylates are the same as the foregoing but the desaminotyrosyl moiety is replaced by a 4-hydroxybenzoyl moiety. In some embodiments, the DT or BT contents include those copolymers with from about 1% to about 30%, from about 5% to about 30% from about 10% to about 30% DT or BT. In some embodiments, the diacids (used informing the polyarylates) include succinate, glutarate and glycolic acid.

In some embodiments, the polymer of coating 24 and/or the polymer of coating 26 includes one or more biodegradable, resorbable polyarylates and polycarbonates. These polymers, include, but are not limited to, BTE glutarate, DTM glutarate, DT propylamide glutarate, DT glycineamide glutarate, BTE succinate, BTM succinate, BTE succinate PEG, BTM succinate PEG, DTM succinate PEG, DTM succinate, DT N-hydroxysuccinimide succinate, DT glucosamine succinate, DT glucosamine glutarate, DT PEG ester succinate, DT PEG amide succinate, DT PEG ester glutarate, DT PEG ester succinate, DTMB P(Desaminotyrsoyl tyrosine methylparaben ester--glutarate), and DTPP P(Desaminotyrsoyl tyrosine propylparaben ester-glutarate).

In some embodiments, the polymer of coating 24 is one more polymers from the **DTE-DT** succinate family of polymers, e.g., the P22-xx family of polymers having from 0-50%, 5-50%, 5-40%, 1-30% or 10-30% DT, including but not limited to, about 1, 2, 5, 10, 15, 20, 25, 27.5, 30, 35, 40%, 45% and 50% DT. In some embodiments, the polymer is P22-27.5 DT.

In some embodiments, the polymer of coating 24 and/or the polymer of coating 26 has diphenol monomer units that are copolymerized with an appropriate chemical moiety to form a polyarylate, a polycarbonate, a polyiminocarbonate, a polyphosphonate or any other polymer.

In some embodiments, the polymer of coating 24 is tyrosine-based polyarylate. In some embodiments, the polymer of coating 24 and/or the polymer of coating 26 includes blends and copolymers with polyalkylene oxides, including polyethylene glycol (PEG).

In some embodiments, the polymer of coating 24 and/or the polymer of coating 26 can have from 0.1-99.9% PEG diacid to promote the degradation process. In some embodiments, the polymer of coating 24 and/or the polymer of coating 26 includes blends of polyarylates or other biodegradable polymers with polyarylates.

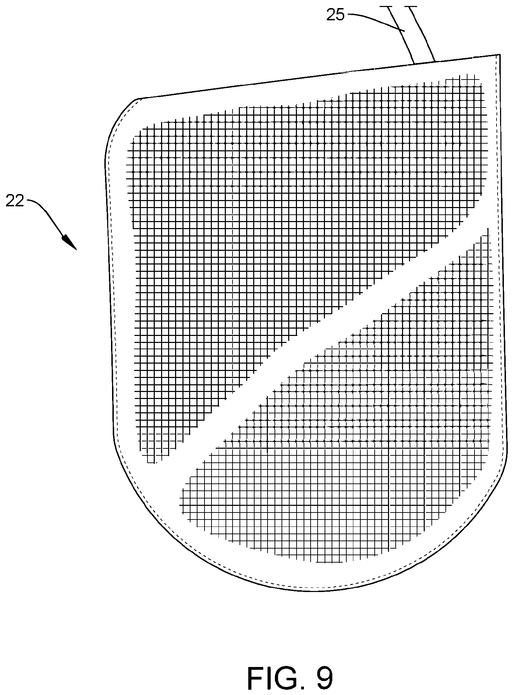

In some embodiments, shown in FIGS. 1, 2, 4, 5 and 9, substrate 22 is a pocket or envelope in which medical device 25 can be at least partially disposed. That is, substrate 22 is a pouch, bag, covering, shell, or receptacle. For example, substrate 22 can include a first piece 22a and a second piece 22b that is joined with first piece 22a. First and second pieces 22a, 22b are joined to form the pocket or envelope. In some embodiments, first and second pieces 22a, 22b are joined along three sides of the pocket or envelope to form a cavity C. First and second pieces 22a, 22b are not joined at a fourth side of the pocket or envelope to define opening O such that medical device 25 can be inserted through opening O and into cavity C to enclose, encase or surround all or a portion of medical device 25 within cavity C. In some embodiments, first and second pieces 22a, 22b are joined with one another along three sides of the pocket or envelope by heat, ultrasonically, bonding, knitting, or adhesive. In some embodiments, the pocket or envelope is monolithically formed by molding the pocket or envelope or producing the pocket or envelope by 3D printing, for example. In some embodiments, anchorage device 20 is pre-formed to provide cavity C with a selected size and shape.

In some embodiments, first and second pieces 22a, 22b are portions of a single sheet that is bent to produce a fold at one end of the pocket or envelope. First and second pieces 22a, 22b are joined along sides of the pocket or envelope that extend transverse to the fold such that the fold and the sides of the pocket or envelope do not have any openings. First and second pieces 22a, 22b are not joined at an end of the pocket or envelope opposite the fold to define an opening at the end such that medical device 25 can be inserted through the opening and into a cavity defined by inner surfaces of first and second pieces 22a, 22b.

In some embodiments, first and second pieces 22a, 22b each include a mesh. In some embodiments, first piece 22a includes a mesh including pores having a first size and second piece 22b includes a mesh including pores having a second size, wherein the first size is different than the second size. In some embodiments, the first size is greater than the second size. In some embodiments, the first size is less than the second size. In some embodiments, first and second pieces 22a, 22b each include a thin walled structure that does not have any pores or apertures. In some embodiments one of first and second pieces 22a, 22b includes a mesh and the other one of first and second pieces 22a, 22b includes a thin walled structure that does not have any pores or apertures.

In some embodiments, first and second pieces 22a, 22b are formed from the same material. In some embodiments one of first and second pieces 22a, 22b is formed from a first material, such as, for example, one or more of the materials discussed herein, and the other one of first and second pieces 22a, 22b is made from a second material, such as, for example, one or more of the materials discussed herein, wherein the second material is different than the first material. For example, first piece 22a may be formed from a biodegradable and/or bioresorbable material and second piece 22b may be formed from a non-biodegradable and/or non-bioresorbable material, or vice versa. In some embodiments, first and second pieces 22a, 22b are each formed from a biodegradable and/or bioresorbable material, wherein the biodegradable and/or bioresorbable materials degrade and/or resorb at the same rate. In some embodiments, first and second pieces 22a, 22b are formed from different biodegradable and/or bioresorbable materials, wherein one of the biodegradable and/or bioresorbable materials degrades and/or resorts more quickly than the other biodegradable and/or bioresorbable material.

In some embodiments, rather include antibiotics and/or other API(s) in a separate coating, such as, for example, coating 24, anchorage device 20 includes a substrate 22 that is formed from a polymer wherein substrate 22 has antibiotics, such as, for example, the antibiotics included in coating 24 and/or other API(s) dispersed in the polymer that forms substrate 22 such that the polymer that forms substrate 22 releases the antibiotics and/or other API(s) as the polymer that forms substrate 22 degrades. Coating 26 then coats all or a portion of substrate 22, as shown in FIG. 10, wherein substrate 22 is made from a polymer with antibiotics and/or other API(s) dispersed within the polymer. In some embodiments, the polymer that forms substrate 22 is a tyrosine-derived polyarylate. In some embodiments, substrate 22 is made at least in part from one or more non-active hemostatic agents that do not initiate or accelerate the clotting process, such as, for example collagen and/or alginates.

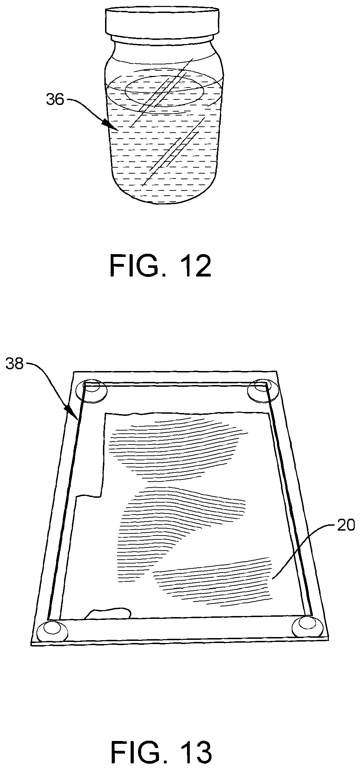

In some embodiments, coating 26 incorporates at least one hemostatic agent, such as, for example, tranexamic acid into a polymer, such as, for example, one or more alginates, in a manner that overcomes the difficulties associated with such hemostatic agents, such as, for example, poor solubility and poor bioavailability. In one example, 2.0 g of glycerol, 3 g of alginate and 2 g of tranexamic acid are added to 300 mL of distilled water to form a mixture 36, as shown in FIG. 12. Mixture 36 is mixed at room temperature until the alginate is fully dissolved. Mixture 36 is then poured into a mold, such as, for example, a 31 cm x 38 cm mold 38. A substrate, such as, for example, substrate 22 is then inserted into mold 38 such that mixture 36 coats substrate 22. Substrate 22 and mixture 36 are then allowed to air dry overnight. Once mixture 36 is dried, substrate 22 and mixture 36 are removed from mold 38. In some embodiments, substrate 22 and mixture 36 are vacuumed dry for four to eight hours.

In operation and use, an anchorage device, such as, for example, anchorage device 20 and a medical device, such as, for example, medical device 25 are implanted into a body of a patient. The anchorage device releases active pharmaceutical ingredients, such as, for example, tranexamic acid and/or other hemostatic agents, to reduce or prevent bleeding within the patient or treat one of the conditions as discussed herein. In some embodiments, anchorage device 20 is implanted within the patient without medical device 25 and medical device 25 is coupled to or inserted into anchorage device 20 after anchorage device 20 is implanted. In some embodiments, medical device 25 is coupled to or inserted into anchorage device 20 before anchorage device 20 is implanted within the patient and anchorage device 20 and medical device 25 are implanted within the patient together.

In some embodiments, medical device 25 is removed from the patient after the treatment is completed. In some embodiments, anchorage device 20 remains implanted within the patient after medical device 25 is removed. In some embodiments, anchorage device 20 is removed from the patient after medical device 25 is removed. To remove anchorage device 20, tissue that is ingrown within substrate 22 of anchorage device 20 can be cut or otherwise detached from substrate 22. In some embodiments, a portion of anchorage device 20 may not be removable from the tissue and will remain implanted within the patient.

Also disclosed are kits that include one or a plurality of anchorage devices, such as, for example, anchorage devices 20. It is contemplated that each of the anchorage devices included can have a different configuration. In some embodiments, the anchorage devices can include different coatings 24 and/or different coatings 26. In some embodiments, the anchorage devices can include different sizes. In some embodiments, the anchorage devices can include different shapes. In some embodiments, the anchorage devices can include different anchorage devices that are designed for use with different medical devices, such as, for example, the implantable or non-implantable medical devices discussed herein. In some embodiments, the kits include one or a plurality of medical devices, such as, for example, the implantable or non-implantable medical devices discussed herein. In some embodiments, the kit includes instructions for use. In some embodiments, the kit includes items that are used to make the anchorage devices, such as, for example, the materials used to make the substrate, the hemostatic agent(s), the active pharmaceutical ingredient(s), a computer with a processor capable of receiving data and communicating with a 3D printer to create an anchorage device having the parameters that were input into the computer (e.g., size, shape, material, agents/ingredients on selected areas of the substrate in selected amounts) and a 3D printer capable of making the anchorage device based upon data that is input into the computer regarding the parameters of the implant.

It will be understood that various modifications may be made to the embodiments disclosed herein. Therefore, the above description should not be construed as limiting, but merely as exemplification of the various embodiments.

## Claims

1. A surgical device comprising:
a substrate;
a first coating that covers at least a portion of the substrate, the first coating comprising a first polymer, the first coating having antibiotics dispersed in the first polymer such that the first polymer releases the antibiotics as the first polymer degrades; and
a second coating that covers at least a portion of the first coating, the second coating comprising a second polymer, the second polymer comprising an alginate, the second coating having a hemostatic agent dispersed in the second polymer such that the second polymer releases the hemostatic agent as the second polymer degrades, the hemostatic agent being selected from the group consisting of epinephrine, tranexamic acid, chitosan and oxidized regenerated cellulose.

2. The surgical device recited in claim 1, wherein the substrate defines a plurality of spaced apart apertures, the first coating directly engaging the substrate without filling the apertures.

3. The surgical device recited in claim 1, wherein the substrate defines a plurality of spaced apart apertures, the first coating directly engaging the substrate without filling the apertures, the second coating directly engaging the first coating such that the first coating and the second coating fill the apertures.

4. The surgical device recited in claim 1, wherein the substrate includes a mesh made from a plurality of fibers, the fibers defining a plurality of spaced apart apertures therebetween, the first coating directly engaging the fibers without filling the apertures such that the first coating defines gaps between adjacent fibers that are coated with the first coating, the second coating filling the gaps.

5. The surgical device recited in claim 1, wherein the substrate includes a mesh made from a plurality of fibers, the first coating directly engaging the fibers such that the first coating extends 360 degrees about each of the fibers along at least a portion of a length of each of the fibers.

6. The surgical device recited in claim 5, wherein the second coating directly engages the first coating such that the second coating extends less than 360 degrees about each of the fibers along at least a portion of the length of each of the fibers.

7. The surgical device recited in claim 5, wherein the second coating directly engaging the first coating such that the second coating extends only between about 200 and 280 degrees about each of the fibers along at least a portion of the length of each of the fibers.

8. The surgical device recited in claim 1, wherein the second coating is spaced apart from the substrate by the first coating.

9. The surgical device recited in claim 1, wherein the substrate is a biodegradable mesh having a first piece and a second piece that is joined with the first piece, the coatings being applied to each of the pieces such that an inner surface of the device that forms a pocket having a cavity and an opening that is in communication with the cavity is defined by the first coating and the second coating and an opposite outer surface of the device is defined solely by the second coating.

10. The surgical device recited in claim 1, wherein the substrate is a biodegradable mesh having a first piece and a second piece that is joined with the first piece, the coatings being applied to each of the pieces such that an inner surface of the device that forms a pocket having a cavity and an opening that is in communication with the cavity is defined solely by the second coating and an opposite outer surface of the device is defined by the first coating and the second coating.

11. The surgical device recited in any one of the above claims, wherein the first polymer has a first degradation rate and the second polymer has a second degradation rate, the first degradation rate being different than the second degradation rate.

12. The surgical device recited in claim 11, wherein the first polymer is a tyrosine-derived polyarylate.

13. The surgical device recited in claim 11, wherein the alginate is selected from the group consisting of calcium alginate and sodium alginate.

14. The surgical device recited in claim 11, wherein:
the second polymer is a tyrosine-derived polyarylate; and
the alginate is selected from the group consisting of calcium alginate and sodium alginate.

15. The surgical device recited in claim 11, wherein the antibiotics are selected from the group consisting of rifampin and minocycline, and mixtures thereof.

## Patentansprüche

1. Chirurgische Vorrichtung umfassend:
ein Substrat;
eine erste Beschichtung, die wenigstens einen Teil des Substrats bedeckt, wobei die erste Beschichtung ein erstes Polymer umfasst, wobei die erste Beschichtung Antibiotika aufweist, die in dem ersten Polymer dispergiert sind, so dass das erste Polymer die Antibiotika freisetzt, wenn sich das erste Polymer abbaut; und
eine zweite Beschichtung, die wenigstens einen Teil der ersten Beschichtung bedeckt, wobei die zweite Beschichtung ein zweites Polymer umfasst, wobei das zweite Polymer ein Alginat umfasst, wobei die zweite Beschichtung ein Hämostatikum aufweist, das in dem zweiten Polymer dispergiert ist, so dass das zweite Polymer das Hämostatikum freisetzt, wenn sich das zweite Polymer abbaut, wobei das Hämostatikum ausgewählt ist aus der Gruppe bestehend aus Epinephrin, Tranexamsäure, Chitosan und oxidierter regenerierter Cellulose.

2. Chirurgische Vorrichtung nach Anspruch 1, wobei das Substrat eine Vielzahl von beabstandeten Öffnungen definiert, wobei die erste Beschichtung direkt mit dem Substrat in Eingriff steht, ohne die Öffnungen zu füllen.

3. Chirurgische Vorrichtung nach Anspruch 1, wobei das Substrat eine Vielzahl von beabstandeten Öffnungen definiert, wobei die erste Beschichtung direkt mit dem Substrat in Eingriff steht, ohne die Öffnungen zu füllen, wobei die zweite Beschichtung direkt mit der ersten Beschichtung in Eingriff steht, so dass die erste Beschichtung und die zweite Beschichtung die Öffnungen füllen.

4. Chirurgische Vorrichtung nach Anspruch 1, wobei das Substrat ein Netz umfasst, das aus einer Vielzahl von Fasern besteht, wobei die Fasern eine Vielzahl von beabstandeten Öffnungen dazwischen definieren, wobei die erste Beschichtung direkt mit den Fasern in Eingriff steht, ohne die Öffnungen zu füllen, so dass die erste Beschichtung Lücken zwischen benachbarten Fasern definiert, die mit der ersten Beschichtung beschichtet sind, wobei die zweite Beschichtung die Lücken füllt.

5. Chirurgische Vorrichtung nach Anspruch 1, wobei das Substrat ein Netz umfasst, das aus einer Vielzahl von Fasern besteht, wobei die erste Beschichtung direkt mit den Fasern so in Eingriff steht, dass die erste Beschichtung 360 Grad um jede der Fasern entlang wenigstens eines Abschnitts einer Länge jeder der Fasern verläuft.

6. Chirurgische Vorrichtung nach Anspruch 5, wobei die zweite Beschichtung direkt mit der ersten Beschichtung so in Eingriff steht, dass die zweite Beschichtung weniger als 360 Grad um jede der Fasern entlang wenigstens eines Abschnitts der Länge jeder der Fasern verläuft.

7. Chirurgische Vorrichtung nach Anspruch 5, wobei die zweite Beschichtung direkt mit der ersten Beschichtung so in Eingriff steht, dass die zweite Beschichtung nur zwischen etwa 200 und 280 Grad um jede der Fasern entlang wenigstens eines Abschnitts der Länge jeder der Fasern verläuft.

8. Chirurgische Vorrichtung nach Anspruch 1, wobei die zweite Beschichtung durch die erste Beschichtung von dem Substrat beabstandet ist.

9. Chirurgische Vorrichtung nach Anspruch 1, wobei das Substrat ein biologisch abbaubares Netz mit einem ersten Stück und einem zweiten Stück, das mit dem ersten Stück verbunden ist, ist, wobei die Beschichtungen auf jedes der Stücke so aufgebracht sind, dass eine Innenfläche der Vorrichtung, die eine Tasche mit einem Hohlraum und einer Öffnung bildet, die mit dem Hohlraum in Verbindung steht, durch die erste Beschichtung und die zweite Beschichtung definiert ist und eine gegenüberliegende Außenfläche der Vorrichtung nur durch die zweite Beschichtung definiert ist.

10. Chirurgische Vorrichtung nach Anspruch 1, wobei das Substrat ein biologisch abbaubares Netz mit einem ersten Stück und einem zweiten Stück, das mit dem ersten Stück verbunden ist, ist, wobei die Beschichtungen auf jedes der Stücke so aufgebracht sind, dass eine Innenfläche der Vorrichtung, die eine Tasche mit einem Hohlraum und einer Öffnung bildet, die mit dem Hohlraum in Verbindung steht, ausschließlich durch die zweite Beschichtung definiert ist und eine gegenüberliegende Außenfläche der Vorrichtung durch die erste Beschichtung und die zweite Beschichtung definiert ist.

11. Chirurgische Vorrichtung nach einem der vorstehenden Ansprüche, wobei das erste Polymer eine erste Abbaurate aufweist und das zweite Polymer eine zweite Abbaurate aufweist, wobei die erste Abbaurate von der zweiten Abbaurate verschieden ist.

12. Chirurgische Vorrichtung nach Anspruch 11, wobei das erste Polymer ein Tyrosin-abgeleitetes Polyarylat ist.

13. Chirurgische Vorrichtung nach Anspruch 11, wobei das Alginat ausgewählt ist aus der Gruppe bestehend aus Calciumalginat und Natriumalginat.

14. Chirurgische Vorrichtung nach Anspruch 11, wobei:
das zweite Polymer ein Tyrosin-abgeleitetes Polyarylat ist; und
das Alginat ausgewählt ist aus der Gruppe bestehend aus Calciumalginat und Natriumalginat.

15. Chirurgische Vorrichtung nach Anspruch 11, wobei die Antibiotika ausgewählt sind aus der Gruppe bestehend aus Rifampin und Minocyclin und Gemischen davon.

## Revendications

1. Dispositif chirurgical comprenant :
un substrat ;
un premier revêtement qui recouvre au moins une partie du substrat, le premier revêtement comprenant un premier polymère, le premier revêtement ayant des antibiotiques dispersés dans le premier polymère de telle sorte que le premier polymère libère les antibiotiques à mesure que le premier polymère se dégrade ; et
un second revêtement qui recouvre au moins une partie du premier revêtement, le second revêtement comprenant un second polymère, le second polymère comprenant un alginate, le second revêtement ayant un agent hémostatique dispersé dans le second polymère de telle sorte que le second polymère libère l'agent hémostatique à mesure que le second polymère se dégrade, l'agent hémostatique étant choisi dans le groupe constitué par épinéphrine, acide tranexamique, chitosane et cellulose régénérée oxydée.

2. Dispositif chirurgical selon la revendication 1, dans lequel le substrat définit une pluralité d'ouvertures espacées les unes des autres, le premier revêtement étant directement en contact avec le substrat sans remplir les ouvertures.

3. Dispositif chirurgical selon la revendication 1, dans lequel le substrat définit une pluralité d'ouvertures espacées les unes des autres, le premier revêtement étant directement en contact avec le substrat sans remplir les ouvertures, le second revêtement étant directement en contact avec le premier revêtement de telle sorte que le premier revêtement et le second revêtement remplissent les ouvertures.

4. Dispositif chirurgical selon la revendication 1, dans lequel le substrat comporte un treillis constitué d'une pluralité de fibres, les fibres définissant une pluralité d'ouvertures espacées entre elles, le premier revêtement étant directement en contact avec les fibres sans remplir les ouvertures de telle sorte que le premier revêtement définisse des espaces entre des fibres adjacentes qui sont revêtues du premier revêtement, le second revêtement remplissant les espaces.

5. Dispositif chirurgical selon la revendication 1, dans lequel le substrat comporte un treillis constitué d'une pluralité de fibres, le premier revêtement étant directement en contact avec les fibres de telle sorte que le premier revêtement s'étende sur 360 degrés autour de chacune des fibres le long d'au moins une partie d'une longueur de chacune des fibres.

6. Dispositif chirurgical selon la revendication 5, dans lequel le second revêtement est directement en contact avec le premier revêtement de telle sorte que le second revêtement s'étende sur moins de 360 degrés autour de chacune des fibres le long d'au moins une partie de la longueur de chacune des fibres.

7. Dispositif chirurgical selon la revendication 5, dans lequel le second revêtement est directement en contact avec le premier revêtement de telle sorte que le second revêtement s'étende seulement entre environ 200 et 280 degrés autour de chacune des fibres le long d'au moins une partie de la longueur de chacune des fibres.

8. Dispositif chirurgical selon la revendication 1, dans lequel le second revêtement est espacé du substrat par le premier revêtement.

9. Dispositif chirurgical selon la revendication 1, dans lequel le substrat est un treillis biodégradable ayant une première pièce et une seconde pièce qui est jointe à la première pièce, les revêtements étant appliqués sur chacune des pièces de telle sorte qu'une surface interne du dispositif qui forme une poche présentant une cavité et une ouverture qui communique avec la cavité soit définie par le premier revêtement et le second revêtement et qu'une surface externe opposée du dispositif soit définie uniquement par le second revêtement.

10. Dispositif chirurgical selon la revendication 1, dans lequel le substrat est un treillis biodégradable ayant une première pièce et une seconde pièce qui est jointe à la première pièce, les revêtements étant appliqués sur chacune des pièces de telle sorte qu'une surface interne du dispositif qui forme une poche présentant une cavité et une ouverture qui communique avec la cavité soit définie par le second revêtement et qu'une surface externe opposée du dispositif soit définie par le premier revêtement et le second revêtement.

11. Dispositif chirurgical selon l'une quelconque des revendications précédentes, dans lequel le premier polymère a une première vitesse de dégradation et le second polymère a une seconde vitesse de dégradation, la première vitesse de dégradation étant différente de la seconde vitesse de dégradation.

12. Dispositif chirurgical selon la revendication 11, dans lequel le premier polymère est un polyarylate dérivé de tyrosine.

13. Dispositif chirurgical selon la revendication 11, dans lequel l'alginate est choisi dans le groupe constitué par alginate de calcium et alginate de sodium.

14. Dispositif chirurgical selon la revendication 11, dans lequel :
le second polymère est un polyarylate dérivé de tyrosine ; et
l'alginate est choisi dans le groupe constitué par alginate de calcium et alginate de sodium.

15. Dispositif chirurgical selon la revendication 11, dans lequel les antibiotiques sont choisis dans le groupe constitué par rifampine et minocycline, et des mélanges de celles-ci.
